# EUROPEAN PATENT APPLICATION

(11) **EP 4 752 224 A1**
(43) Date of publication of application: **03.06.2026**
(21) Application number: 24845693.1
(22) Date of filing: 26.07.2024
(51) Int. Cl.: C12N 15/35, C07K 7/06, C07K 14/015, C12N 1/15, C12N 1/19, C12N 1/21, C12N 5/10, C12N 7/00, C12N 7/01, C12N 15/864

(54) **BRAIN-TROPIC AAV MUTANT**

(30) Priority: 27.07.2023 JP 2023122666; 05.10.2023 JP 2023173364; 05.02.2024 JP 2024015494
(71) Applicant: Takara Bio Inc., Kusatsu-shi Shiga 525-0058 (JP)
(72) Inventor: TANAKA, Yoshinori, Kusatsu-shi, Shiga 525-0058 (JP); KOHARA, Sayuri, Kusatsu-shi, Shiga 525-0058 (JP); OKUDA, Nozomi, Kusatsu-shi, Shiga 525-0058 (JP); ISHIKAWA, Toshiko, Kusatsu-shi, Shiga 525-0058 (JP); OKAMOTO, Sachiko, Kusatsu-shi, Shiga 525-0058 (JP); TANAKA, Rina, Kusatsu-shi, Shiga 525-0058 (JP)
(74) Representative: Müller, Christian Stefan Gerd
(86) International application number: PCT/JP2024/026797
(87) International publication number: WO 2025/023319

(57) **Abstract**

The present invention provides a nucleic acid encoding an adeno-associated virus (AAV) capsid protein mutant which includes a peptide comprising an amino acid sequence selected from the group consisting of SEQ ID NO: 1 to SEQ ID NO: 4.

## Description

### TECHNICAL FIELD

The present invention relates to a mutant of an adeno-associated virus (AAV) capsid protein, the capsid protein mutant having high tropism for brain and low tropism for lung, a nucleic acid encoding the capsid protein mutant, an AAV particle comprising the capsid protein mutant, and a method of producing a gene-transduced cell by use of the particle.

### BACKGROUND ART

AAV is a virus having a linear single-stranded DNA genome of 4.7 kb, comprising open reading frames of two genes rep and cap. The rep gene encodes four proteins necessary for genome replication (Rep78, Rep68, Rep52, and Rep40). The cap gene encodes three capsid proteins that assemble for formation of a viral capsid (VP1, VP2, VP3), and assembly-activating protein (AAP). Replication of AAV in nature relies on the presence of a helper virus such as an adenovirus or a herpes virus. In the absence of a helper virus, the genome of AAV is maintained in an episome or integrated into a chromosome of a host, so that the AAV is present in a latent state. Over one hundred serotypes and clades (non-patent literature 1) of AAV are currently identified. Particularly, development of vectors for gene delivery based on AAV2 is advanced.

In 1989, a gene delivery vector system based on AAV2 was developed for the first time. Vectors based on AAV have been found to have many advantages. Since wild-type AAV is nonpathogenic and has no etiological relation to any known diseases, vectors based on AAV are believed to be extremely safe. In addition, AAV has high gene transduction efficiency.

Administration of AAV particles enables long-period and stable gene transduction into various target organs and target cells. Until now, gene transduction with high efficiency into skeletal muscles, liver (hepatic cells), heart (cardiac muscle cells), nerve cells, pancreatic gland cells, and pancreatic islet cells has been reported. In addition, AAV has been used in human clinical trials. On the other hand, an attempt to change the cell tropism of AAV by alteration of capsid proteins of the AAV and an attempt to avoid removal of AAV particles by neutralizing antibodies have been made. For example, AAV capsids with tropism for specific organs and cells such as neuroglia cells, airway epithelial cells, coronary artery vascular endothelial cells, and lung, and AAV capsids with tropism for tumor cells such as glioblastoma cells, melanoma cells, lung cancer cells, and breast cancer cells have been created (non-patent literature 2).

In recent years, it has been shown that AAV9 vectors and their modified type vectors can cross the blood-brain barrier to deliver target genes to the brain, and it has been reported that intravascular administration of AAV9 vectors and their modified type vectors can introduce genes into nerve cells (neurons) throughout the entire brain in mice.

For example, WO2015/038958 (Patent Document 1) discloses AAV vectors containing specific amino acid sequences. Among these, a vector in which the amino acid sequence TLAVPFK was inserted at position 588 of the AAV9 capsid protein was designated AAV-PHP.B. AAV-PHP.B was shown to transfer genes throughout the CNS after intravenous administration with efficiency at least 40 times higher than that of AAV9 (Non-Patent Document 3).

Furthermore, AAV-PHP.eB, which contains the amino acid sequence DGTLAVPFK inserted at position 588 of the AAV9 capsid protein, has been shown to reduce the amount of virus required to transduce most CNS neurons (Non-Patent Document 5). Furthermore, it has been shown that systemic and intravitreal delivery of AAV-PHP.eB results in high transduction efficiency into retinal ganglion cells and horizontal cells (Non-Patent Document 6).

Furthermore, WO2015/158749 (Patent Document 2) discloses a capsid protein for an AAV vector, the capsid protein containing a specific amino acid sequence that specifically binds to cells of the brain and/or spinal cord. It has been shown that an AAV vector specific to brain microvascular endothelial cells can be produced by inserting a consensus sequence (XXGXXWX) consisting of seven amino acids at position 588 of an AAV2 capsid protein (Non-Patent Document 4).

Furthermore, WO2020/218419 (Patent Document 3) discloses a mutant of an AAV capsid protein, the mutant containing a specific amino acid sequence and having brain tropism.

However, since AAV vectors are generally administered systemically, AAV vectors with higher brain-tropism are needed for gene therapy targeting brain diseases.

### PRIOR ART DOCUMENT

### PATENT DOCUMENT

Patent Document 1: WO2015/038958
Patent Document 2: WO2015/158749
Patent Document 3: WO2020/218419

### NON-PATENT DOCUMENT

Non-patent Document 1: Gao et al., J. Virology, Vol. 78, pp. 6381-6388, 2004
Non-patent Document 2: Adachi K. et al., Gene Ther. Regul., Vol. 5, pp. 31-55, 2010
Non-patent Document 3: Nat Biotechnol. 2016 Feb;34(2): 204-9
Non-patent Document 4: EMBO Mol. Med., 2016 Jun 1;8(6): 609-25
Non-patent Document 5: Nat. Neurosci. 2017 Aug;20(8): 1172-1179
Non-patent Document 6: Mol. Ther. Methods Clin. Dev. 2022 Mar 28;25: 236-249

### SUMMARY OF THE INVENTION

### PROBLEMS TO BE SOLVED BY THE INVENTION

Objections of the present invention includes provision of an AAV capsid protein mutant with high tropism for brain and low tropism for lung, and provision of a method of efficiently introducing a gene into a brain.

### SOLUTIONS TO THE PROBLEMS

The present inventors intensively made efforts to solve the above-described problems, and as a result, created an AAV capsid protein mutant comprising a peptide containing an amino acid sequence selected from the group consisting of SEQ ID NOs: 1-4. Thus the present invention was completed.

The present invention generally relates to:
[1] A nucleic acid encoding a mutant of an adeno-associated virus (AAV) capsid protein which comprises a peptide comprising an amino acid sequence selected from the group consisting of SEQ ID NOs: 1-4;
[2] The nucleic acid according to [1], wherein the AAV capsid protein is derived from AAV2;
[3] The nucleic acid according to [2], wherein the peptide is placed at a position between amino acid number 588 and amino acid number 589 in VP1 of AAV2;
[4] A recombinant DNA comprising the nucleic acid according to any one of [1] to [3];
[5] A cell comprising the nucleic acid according to any one of [1] to [3];
[6] An AAV particle comprising a mutant of an AAV capsid protein which comprises a peptide comprising an amino acid sequence selected from the group consisting of SEQ ID NOs: 1-4;
[7] The AAV particle according to [6], wherein the AAV capsid protein is derived from AAV2;
[8] The AAV particle according to [7], wherein the peptide is placed at a position between amino acid number 588 and amino acid number 589 in VP1 of AAV2;
[9] A method of producing a gene-transduced cell, the method comprising a step of bringing an AAV particle comprising a mutant of an AAV capsid protein which comprises a peptide comprising an amino acid sequence selected from the group consisting of SEQ ID NOs: 1-4 into contact with a cell;
[10] The method according to [9], wherein the AAV capsid protein is derived from AAV2;
[11] The method according to [10], wherein the peptide is placed at a position between amino acid number 588 and amino acid number 589 in VP1 of AAV2;
[12] A method of producing an AAV particle, the method comprising using the cell according to [5] as a host.

### EFFECTS OF THE INVENTION

According to the present invention, a gene transduction system useful for gene transduction into brain is provided. The AAV particle of the present invention has high cell tropism for brain and low cell tropism for lung, and thus a gene transduced by the AAV particle can be strongly expressed in the brain.

### BRIEF DESCRIPTION OF THE DRAWINGS

[Fig. 1] Figure 1 shows a method of producing a nucleic acid construct for enabling a capsid protein to comprise a random peptide.
[Fig. 2] Figure 2 shows the appearance frequency of AAV vectors in each tissue in Example 4.
[Fig.3] Figure 3 shows measurement results of luciferase activity in Example 5.
[Fig. 4] Figure 4 shows results of fluorescence microscopic measurement of AcGFP in Example 6.

### MODE FOR CARRYING OUT THE INVENTION

As used herein, the "adeno-associated virus" refers to a small virus belonging to the genus Dependovirus which lies within the family Parvoviridae and capable of infecting primates including human and other mammals. Hereinafter, the adeno-associated virus is abbreviated as AAV. AAV has a non-enveloped shell (capsid) of a regular icosahedron and a linear single-stranded DNA inside the shell. As used herein, AAV includes the wild-type virus and derivatives thereof, and includes all serotypes and clades of AAV unless specified otherwise.

The "vector" as used herein means a molecule or an associated molecule that is used for mediating delivery of a polynucleotide to a cell and which comprises the polynucleotide or associates with the polynucleotide. Examples of the vector include vector DNAs such as plasmid vectors and phage vectors, viral vector particles, liposomes, and other vehicles for gene delivery, unless specified otherwise.

The "capsid protein" as used herein means a protein that is encoded by the cap gene present in the genome of AAV and constitutes the capsid of AAV. The wild-type AAV genome encodes three capsid proteins, and there are VP1, VP2 and VP3. As used herein, the capsid protein includes VP1, VP2 and VP3. The capsid protein is sometimes referred to as the "Cap protein".

### (1) Nucleic acid encoding an AAV capsid protein mutant

The nucleic acid of the present invention encodes a mutant of an AAV capsid protein which comprises a peptide comprising an amino acid sequence selected from the group consisting of SEQ ID NOs: 1-4. Preferably, the nucleic acid of the present invention encodes an AAV capsid protein mutant that comprises a peptide comprising an amino acid sequence of SEQ ID NO: 1.

The AAV capsid protein mutant encoded by the nucleic acid of the present invention can be prepared by inserting the peptide into an AAV capsid protein of any wild-type AAV, such as AAV type 1 (AAV1), AAV type 2 (AAV2), AAV type 3 (AAV3A, AAV3B etc.), AAV type 4 (AAV4), AAV type 5 (AAV5), AAV type 6 (AAV6), AAV type 7 (AAV7), AAV type 8 (AAV8), AAV type 9 (AAV9), AAV type 10 (AAV10), AAV type 11 (AAV11), avian AAV, bovine AAV, canine AAV, equine AAV, or ovine AAV, or replacing a part of the amino acid sequence of the AAV capsid protein with the peptide (in other words, by making the AAV capsid protein comprise the peptide). In the present invention, a capsid protein of AAV2 is preferably used. The amino acid sequence of the VP1 capsid protein of wild-type AAV2 is shown in SEQ ID NO: 9 (GenBank: AAC03780.1).

The AAV capsid protein mutant encoded by the nucleic acid of the present invention may be a protein comprising substitution, deletion, insertion and/or addition of one or more or several amino acids as well as the above-mentioned peptide in the wild-type AAV capsid protein. The "protein comprising substitution, deletion, insertion and/or addition of one or more or several amino acids as well as the above-mentioned peptide" retains the properties of the original protein, for example the cell tropism of the AAV capsid protein mutant conferred by the above-mentioned peptide, the capsid-forming ability, the function of capsid protein (for example, protection of viral genome, uncoating after entry into host cells) and the like. In the present invention, for example, an AAV2 capsid protein in which asparagine at position 587 is replaced with glutamine is used. The amino acid sequence of the AAV2 VP1 capsid protein comprising glutamine at position 587 is shown in SEQ ID NO:10.

Further, a spacer may be added to the N terminal and/or C terminal of the peptide to be comprised in the AAV capsid protein. The spacer preferably consists of 1 to 5 amino acid residues. The amino acid residues constituting the spacer are not particularly limited. For example, the spacer may comprise an amino acid selected from the group consisting of glycine, alanine and serine. A single amino acid residue of glycine is suitable as a spacer added to the N-terminus, and a single amino acid residue of alanine is suitable as a spacer added to the C-terminus.

As the AAV capsid protein for comprising the peptide, AAV VP1, VP2 or VP3 may be used. Only any one of VP1, VP2 and VP3 may be made to comprise the peptide, or all of VP1, VP2 and VP3 may be made to comprise the peptide. Furthermore, two capsid proteins such as VP1 and VP2, VP2 and VP3, or VP1 and VP3 may be made to comprise the peptide. VP1 to VP3 are encoded by the cap gene region in the AAV genome. In one embodiment of the present invention, a region shared by VP1 to VP3 is made to comprise the peptide so that a mutation can be introduced into all of VP1 to VP3. In another embodiment of the present invention, a gene encoding VP1, VP2 or VP3 is prepared separately from the cap gene region of AAV, and a mutation is introduced into the gene. In this case, a treatment that inhibits a wild-type capsid protein corresponding to a capsid protein encoded by the gene into which a mutation has been introduced from being expressed from the cap gene region of AAV may be performed.

In the case where AAV2 VP1 is used, the AAV capsid protein mutant encoded by the nucleic acid of the present invention preferably comprises the peptide at a position between amino acid number 588 and amino acid number 589. The amino acid number 588 of AAV2 VP1 is arginine. The amino acid number 589 of AAV2 VP1 is glutamine. The amino acid number 588 of AAV2 VP1 corresponds to the amino acid number 451 of AAV2 VP2 and the amino acid number 386 of AAV2 VP3. In the case where a capsid protein of AAV serotypes and clades other than AAV2 is used as the AAV capsid protein, the AAV capsid protein is made to comprise the peptide between amino acids corresponding to amino acid numbers 588 and 589 of AAV2 VP1. A person skilled in the art can easily identify an amino acid of a capsid protein of AAV serotypes and clades other than AAV2 which corresponds to the amino acid at amino acid number 588 of AAV2 VP1. For example, see an alignment of amino acid sequences of VP1 shown in Gao et al., Proc. Natl. Acad. Sci. USA, Vol.99, No.18, pp. 11854-11859, 2002. For example, the amino acid number 588 of AAV2 VP1 corresponds to the amino acid number 589 of AAV1, the amino acid number 590 of AAV7, and the amino acid number 591 of AAV8.

In the present invention, an AAV capsid protein mutant (e.g., SEQ ID NO: 12) containing a spacer(s) and ETGHGYV (SEQ ID NO: 1) between amino acid positions 588 and 589 of the AAV2 VP1 capsid protein is preferably used.

The nucleic acid of the present invention may be operably linked to a suitable control sequence. Examples of the control sequence include a promoter sequence, a polyadenylation signal, a transcription termination sequence, an upstream regulatory domain, a replication origin, an internal ribosomal entry site (IRES), and an enhancer. Examples of the promoter sequence include an inducible promoter sequence, and a constitutive promoter sequence. The control sequence may be an endogenous or exogenous sequence of AAV from which the capsid protein originates, a native sequence, or an artificially designed sequence. The present invention also includes such a recombinant DNA capable of expressing the AAV capsid protein mutant.

The recombinant DNA of the present invention is useful for delivering the nucleic acid of the present invention to cells in vitro, ex vivo or in vivo and imparting the ability to express the AAV capsid protein mutant to the cells. Then, the cell to which the nucleic acid of the present invention is delivered is useful for producing AAV particles. The recombinant DNA can be particularly used for delivery or introduction of the nucleic acid of the present invention into animal cells, preferably mammal cells.

In the present invention, the recombinant DNA of the present invention can be prepared by making a DNA used as a vector retain the nucleic acid of the present invention. For example, a plasmid DNA, a phage DNA, a transposon, a cosmid DNA, an episomal DNA, or a viral genome can be used.

### (2) Cell containing the nucleic acid of the present invention

The present invention also provides a cell comprising the nucleic acid of the present invention, specifically an isolated host cell containing the recombinant DNA as described in above (1). An isolated cell is, for example, a cell line maintained in vitro. The host cell of the present invention is useful for production of the AAV particle of the present invention, as explained below. When the host cell of the present invention is used for producing AAV particles, the host cell may be referred to as a "packaging cell" or "producer cell". The host cell of the present invention may comprise the recombinant DNA of the present invention as described in above (1) integrated into the genome, or retain the recombinant DNA in the cell so as to transiently express the AAV capsid protein mutant.

Introduction of the recombinant DNA of the present invention into a host cell can be performed by a known method. For example, electroporation, calcium phosphate precipitation, direct microinjection into cells, liposome-mediated gene transfection, or nucleic acid delivery using a high-speed particle gun can be used. When a viral vector is used, an infection method suitable for the vector may be selected. By use of such an established technique, the recombinant DNA of the present invention is introduced stably into a chromosome of a host cell or transiently into a cytoplasm of a host cell. For stable transformation, a selectable marker, for example a well-known selectable marker such as a neomycin resistance gene (encoding neomycin phosphotransferase), or a hygromycin B resistance gene (encoding aminoglycoside phosphotransferase (APH)) may be linked to the recombinant DNA of the present invention.

As the host cell, various cells, for example, mammal cells including mouse cells and primate cells (for example, human cells) or insect cells can be used. Suitable examples of mammal cells include, but not limited to, primary cells and cell lines. Examples of suitable cell lines include 293 cells, COS cells, HeLa cells, Vero cells, 3T3 mouse fibroblasts, C3H10T1/2 fibroblasts, CHO cells, and cells derived from them.

### (3) AAV particle comprising an AAV capsid protein mutant encoded by the nucleic acid of the present invention

The AAV particle of the present invention is an AAV particle comprising an AAV capsid protein mutant comprising a peptide comprising an amino acid sequence selected from the group consisting of SEQ ID NO: 1 to SEQ ID NO: 4, encoded by the nucleic acid as described in above (1). The AAV particle of the present invention can be produced from the host cell described in above (2). The AAV particle of the present invention has tropism for a brain, and is useful for gene introduction into a brain. The AAV particle of the present invention also has tropism for a spinal cord. Since the brain and spinal cord are collectively called the central nervous system, it can also be said that the AAV particle of the present invention has tropism for the central nervous system. The brain and spinal cord include cells such as neuronal cells and glial cells (microglia, oligodendrocytes, astrocytes). The gene introduced by the AAV particle of the present invention is strongly expressed in the above-mentioned tissues, organs and cells.

For production of the AAV particle, a cell comprising some elements necessary for production of AAV particles can be used as a packaging cell. The first element is a vector genome (also referred to as an expression vector) for a recombinant AAV which may be replicated in a host cell and packaged in an AAV particle. The recombinant AAV vector genome comprises a heterologous polynucleotide of interest, and AAV inverted terminal repeat (ITR) sequences located on each side, i.e. 5'- and 3'-sides of the heterologous polynucleotide of interest. The heterologous polynucleotide of interest may have a control sequence for the expression. The nucleotide sequences of ITR sequences are known. For AAV2-ITR sequences, for example, see Human Gene Therapy, Vol.5, pp. 793-801, 1994. As the AAV ITR sequences, ITR sequences derived from any of various AAV serotypes including AAV1, AAV2, AAV3, AAV4, AAV5, AAV7 and the like can be used. The ITR sequences used in the present invention may be derived from a wild-type AAV or may be altered by insertion, deletion or substitution of a nucleotide(s). The ITR sequences enable replication of the recombinant AAV vector genome in the presence of Rep protein, and enable incorporation of the recombinant AAV vector genome into a capsid particle in the formation of an AAV particle.

The size of the heterologous polynucleotide of interest which can be harbored inside the AAV particle of the present invention is generally less than about 5 kilo bases (kb). The heterologous polynucleotide of interest may be, for example, a gene encoding a protein of interest which a recipient lacks or loses, a gene encoding a protein having a desired biological or therapeutic activity (for example, antimicrobial, antiviral, or antitumor activity), a desired nucleotide sequence encoding RNA that inhibits or decreases production of a harmful or undesired protein, or a nucleotide sequence encoding an antigenic protein. The heterologous polynucleotide of interest can be appropriately selected according to purposes.

In one embodiment of the present invention, the recombinant AAV vector genome lacks the cap gene region and/or the rep gene region. In this embodiment, an AAV particle into which the recombinant AAV vector genome is packaged is not replicated alone to form an AAV particle again in an infected cell.

The second element necessary for production of AAV particles is a construct that provides proteins encoded in the wild-type AAV. The construct encodes AAV-derived genes providing AAV gene products required for formation of AAV particles. In other words, the construct comprises one or both of the major AAV ORFs, coding regions of the rep gene region and cap gene region. For production of the AAV particle of the present invention, at least a nucleic acid encoding an AAV capsid protein mutant comprising a peptide comprising an amino acid sequence selected from the group consisting of SEQ ID NO: 1 to SEQ ID NO: 4 is used as the cap gene. The host cell of the present invention described in above (2) which is capable of expressing the mutant can be used for production of the AAV particle. The AAV particle has a shell composed of many capsid proteins. All of the capsid proteins may be mutants, or a part of the capsid proteins may be mutants and the others may be wild-type capsid proteins. The AAV particle of the present invention may comprise one kind of a capsid protein mutant or plural kinds of a capsid protein mutants.

The rep gene of AAV is contained in coding regions of the rep gene, and includes genes encoding replication proteins Rep78, Rep68, Rep52 and Rep40. These Rep expression products are shown to possess many functions, including recognition, binding and nicking of the AAV genomic DNA replication origin, DNA helicase activity, and modulation of transcription from AAV-derived promoters.

The third element necessary for production of AAV particles is helper virus functions (also referred to as accessary functions) for AAV replication. For introduction of the helper functions, an adenovirus is generally used. However, other viruses such as herpes simplex virus type-1 or type-2, and vaccinia virus can be also used. When a virus is used, a host cell is infected with the virus as a helper virus. For example, since expression of adenovirus early genes is only required for packaging of AAV particles, an adenovirus that does not reveal expression of late genes may be used. An adenovirus mutant lacking late gene expression (for example, ts100K or ts149 adenovirus variant) can be also used. A nucleic acid construct that provides helper virus functions can be also prepared by use of nucleic acids necessary for the helper virus functions isolated from a helper virus, and then can be introduced into a host cell. The construct that provides the helper virus functions comprises a nucleotide sequence providing one or plural helper virus functions, and is provided to a host cell in the form of a plasmid, phage, transposon, cosmid, or other viruses.

For production of AAV particles, (a) a step of introducing the first element, the recombinant AAV vector genome into a host cell, (b) a step of introducing the second element, the construct that provides AAV helper functions into the host cell, and (c) a step of introducing the third element, the helper virus functions into the host cell are performed. These steps may be performed at the same time, or may be performed in order. The order of steps (a) to (c) may be any order. When the first to third elements are introduced into a host cell, the rep gene-expression products excise and replicate the recombinant vector genome. The capsid proteins expressed form a capsid, and the recombinant vector genome is packaged in the capsid to produce an AAV particle. When the host cell expresses an AAV capsid protein mutant, the shell of the AAV particle produced comprises the AAV capsid protein mutant.

The AAV particle can be isolated and purified from a culture supernatant or a lysate of the host cell by various purification methods such as CsCl density-gradient centrifugation. When a virus is used in above-described step (c), for example, a step of separating the AAV particle from the helper virus on the basis of their size may be added. The AAV particle can be also separated from the helper virus on the basis of a difference in affinity for heparin. Furthermore, the remaining helper viruses can be inactivated by known methods. For example, adenoviruses can be inactivated by heating at about 60°C, for example, for 20 minutes or more. Since AAV particles are very stable to heat, the above-described treatment is effective for selective removal of adenoviruses used as the helper virus.

### (4) Method of producing a gene-transduced cell of the present invention

The AAV particle of the present invention obtained by above (3) is used for delivery of a heterologous polynucleotide of interest to a cell for the purpose of gene therapy or other purposes. The AAV particle is generally introduced into a cell in vivo or in vitro. For in vitro introduction, the AAV particle is brought into contact with a cell obtained from a living body. Then, the cell can be also administered to a living body. For administration of the cell to a living body, the cell can be formulated as a pharmaceutical composition, and various techniques such as intramuscular, intravenous, subcutaneous and intraperitoneal administration can be used. For in vivo transduction, the AAV particle is formulated as a pharmaceutical composition, and in general, administrated parenterally (for example, administered via an intramuscular, subcutaneous, intratumor, transdermal, or intraspinal route). The pharmaceutical composition comprising the AAV particle may contain a pharmaceutically acceptable carrier and, as necessary, other drug, medicine, stabilizer, carrier, adjuvant, diluent, and the like.

The pharmaceutical composition comprising the AAV particle of the present invention can be used to deliver a desired polynucleotide, for example a polynucleotide effective in the prevention or treatment of disease, to a cell, tissue, or organ which the AAV particle has tropism for. In other words, the pharmaceutical composition is useful as a preventive or therapeutic agent for disease. Furthermore, a method for preventing or treating a disease, the method comprising using the pharmaceutical composition is also provided an aspect of the present invention.

### EXAMPLES

Hereinafter, the present invention is explained with reference to Examples which the present invention is not particularly limited to.

### Example 1: Preparation of recombinant plasmids

### (1) pRC2-Insert

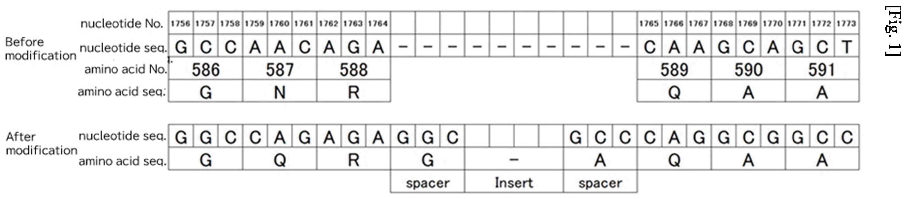

Multiple plasmids containing various amino acid sequences inserted between amino acid numbers 588 and 589 of AAV2 Cap protein were prepared by modifying plasmid pRC2 (manufactured by Takara Bio Inc.) containing sequences encoding AAV2 Rep protein and AAV2 Cap protein. Figure 1 shows the nucleotide sequences before and after modification of positions 1756 to 1773 of the AAV2 cap gene and the amino acid sequences before and after modification of positions 586 to 591 of the AAV2 Cap protein. The multiple plasmids thus obtained were collectively named pRC2-Insert. The names of plasmids included in pRC2-Insert and the inserted amino acid sequences (excluding spacers) are shown in the first column and the second column of Tables 1 to 3, respectively.

### (2) pAAV-CAG-AcGFP-BC

An oligo DNA (SEQ ID NO: 5: taaggatccgcacnnnnnnnnnnnnnnnctggggatccacgggtggcat (wherein, n represents a, t, g, or c) containing a random 15-nucleotide sequence (hereinafter referred to as a barcode sequence) was artificially synthesized. The oligo DNA, a primer (SEQ ID NO: 6), and Klenow Fragment (3'→5' exo-) (manufactured by NEB) were mixed and reacted at 37°C for 1 hour and 30 minutes. This reaction produced a double-stranded DNA from the oligo DNA as a template. The double-stranded DNA was purified using NucleoSpin (registered trademark) Gel and PCR Clean-up (manufactured by Machley-Nagel) and then digested with restriction enzyme BamHI (manufactured by Takara Bio). The double-stranded DNA was inserted downstream of the termination codon of an AcGFP gene in pAAV-CAG-AcGFP previously digested with BamHI, using DNA Ligation Kit <Mighty Mix> (manufactured by Takara Bio Inc.). The multiple plasmids thus obtained were collectively designated pAAV-CAG-AcGFP-BC. After cloning the plasmids included in pAAV-CAG-AcGFP-BC, the barcode sequence contained in each plasmid was identified by Sanger sequencing. The names of plasmids included in pAAV-CAG-AcGFP-BC are shown in the third column of Tables 1 to 3.

### Example 2: Production of AAV vectors

### (1) Seeding of 293T cells

293T cells were suspended in DMEM (manufactured by Sigma) containing 10% FBS and 50 µg/ml streptomycin sulfate solution and seeded into approximately 90 T225 cell culture flasks (manufactured by Corning) at 3 × 10⁶ cells/flask. The 293T cells were cultured in a CO₂ incubator at 37°C for 72 hours.

### (2) Plasmid introduction into 293T cells

In each of approximately 90 culture vessels, the 293T cells were transfected with one plasmid of pRC2-Insert, one plasmid of pAAV-CAG-AcGFP-BC, and pHelper Vector (manufactured by Takara Bio Inc.) using PEIpro (registered trademark) (manufactured by Polyplus transfection). Combinations of pRC2-Insert and pAAV-CAG-AcGFP-BC for transfection are shown in Tables 1 to 3. The 293T cells were cultured in a CO₂ incubator at 37°C for 72 hours.

**[Table 1]**

| pRC2-Insert | amino acid sequence | pAAV-CAG-AcGFP-BC |
|---|---|---|
| pRC2 | ------- | BC1 |
| | | BC2 |
| | | BC3 |
| Insert1 | ETGHGWV | BC4 |
| | | BC5 |
| | | BC6 |
| Insert2 | ATGHGWV | BC7 |
| | | BC8 |
| Insert3 | EAGHGWV | BC9 |
| | | BC10 |
| Insert4 | ETAHGWV | BC11 |
| | | BC12 |
| Insert5 | AAGHGWV | BC13 |
| | | BC14 |
| Insert6 | ATAHGWV | BC15 |
| | | BC16 |
| Insert7 | EAAHGWV | BC17 |
| | | BC18 |
| Insert8 | ETGAGWV | BC19 |
| | | BC20 |
| Insert9 | ETGHAWV (SEQ ID NO:3) | BC21 |
| | | BC22 |
| Insert10 | ETGAAWV | BC23 |
| | | BC24 |
| Insert11 | ETGHGAV | BC89 |
| | | BC26 |
| Insert 12 | ETGHGYV (SEQ ID NO:1) | BC27 |
| | | BC28 |
| Insert 13 | ETGHGFV (SEQ ID NO:2) | BC29 |
| | | BC30 |
| Insert 14 | ETGHGWA (SEQ ID NO:4) | BC31 |
| | | BC32 |

**[Table 2]**

| pRC2-Insert | amino acid sequence | pAAV-CAG-AcGFP-BC |
|---|---|---|
| Insert 15 | ETGHGAA | BC33 |
| | | BC34 |
| Insert 16 | ATGAGWV | BC35 |
| | | BC36 |
| Insert17 | ATGHAWV | BC37 |
| | | BC38 |
| Insert 18 | ATGHGAV | BC39 |
| | | BC40 |
| Insert19 | ATGHGWA | BC41 |
| | | BC42 |
| Insert20 | EAGAGWV | BC43 |
| | | BC44 |
| Insert21 | EAGHAWV | BC45 |
| | | BC46 |
| Insert22 | EAGHGAV | BC47 |
| | | BC48 |
| Insert23 | EAGHGWA | BC49 |
| | | BC50 |
| Insert24 | ETAAGWV | BC51 |
| | | BC52 |
| Insert25 | ETAHAWV | BC53 |
| | | BC54 |
| Insert26 | ETAHGAV | BC55 |
| | | BC56 |
| Insert27 | ETAHGYV | BC57 |
| | | BC58 |
| Insert28 | ETAHGFV | BC59 |
| | | BC60 |

**[Table 3]**

| pRC2-Insert | amino acid sequence | pAAV-CAG-AcGFP-BC |
|---|---|---|
| Insert29 | ETAHGWA | BC61 |
| | | BC62 |
| Insert30 | ETGAGAV | BC63 |
| | | BC64 |
| Insert31 | ETGAGWA | BC65 |
| | | BC66 |
| Insert32 | ETGHAAV | BC67 |
| | | BC68 |
| Insert33 | ETGHAWA | BC69 |
| | | BC70 |
| Insert34 | AAAHGWV | BC71 |
| | | BC72 |
| Insert35 | AAAAGWV | BC73 |
| | | BC74 |
| Insert36 | AAAAAWV | BC75 |
| | | BC76 |
| Insert37 | AAAAAAV | BC77 |
| | | BC78 |
| Insert38 | AAAAAAA | BC79 |
| | | BC80 |
| Insert39 | EAAAAAA | BC81 |
| | | BC82 |
| Insert40 | ETAAAAA | BC83 |
| | | BC84 |
| Insert41 | ETGAAAA | BC85 |
| | | BC86 |
| Insert42 | ETGHAAA | BC87 |
| | | BC88 |

### (3) Solubilization of 293T cells and recovery of supernatant

In each of approximately 90 culture vessels, the 293T cells were suspended in a solution containing a surfactant and then solubilized. Each suspension was collected in a 50 mL centrifuge tube and centrifuged, and then a supernatant was collected. The collected supernatant was frozen and stored at -80° C until use.

### (4) Measurement of viral genome quantity

The quantity of viral genomes contained in the supernatant recovered in Example 2-(3) was measured using AAVpro (registered trademark) Titration Kit Ver. 2 (manufactured by Takara Bio Inc.).

### Example 3: Purification of AAV Vector

### (1) Affinity Chromatography

Approximately 90 supernatants that had been frozen and stored in Example 2-(3) were thawed, and the supernatants were mixed at 5 x 10¹¹ vg each. The mixture was filtered using a 0.45 µm bottle-top filter (manufactured by Corning) and then applied to AVB Sepharose High Performance (manufactured by Cytiva). An eluate was separated into 1 mL fractions, and the amount of viral genomes contained in each fraction was measured using the same method as in Example 2-(4). Each fraction was subjected to protein quantification by A280 measurement. As a result, fractions enriched in AAV vectors were found.

### (2) Cesium chloride density gradient centrifugation

After mixing the fractions enriched in AAV vectors, a cesium chloride solution was added to adjust the refractive index to 1.371. The mixture was dispensed into ultracentrifuge 13PA tubes (manufactured by Eppendorf Himac Technologies) and centrifuged at 34,000 rpm and 21° C for 42 hours. After centrifugation, the supernatant was fractionated into 0.5 mL fractions from the top of the tube. Using the same method as in Example 3-(1), fractions enriched in AAV vectors were found.

### (3) Dialysis

The fractions enriched in AAV vectors obtained in Example 3-(2) were mixed and then subjected to a Slide-A-lyzer dialysis cassette (manufactured by Thermo Scientific). The dialysis cassette was immersed in 1 L of a phosphate buffered saline (PBS), and dialysis was carried out at 4°C for 2 hours or more three times. Next, the dialysis cassette was immersed in 1 L of a PBS containing 0.001% Pluronic F-68 (manufactured by Thermo Scientific), 5% sorbitol and 200 mM NaCl, and dialyzed at 4°C for 3 hours. A solution was recovered from the dialysis cassette, and concentrated by ultrafiltration using an Amicon (registered trademark) Ultra-4 Centrifugal Filter Unit (manufactured by Merck). The concentrated solution was filtered using a 0.22 µm filter (manufactured by Millipore) and stored at -80° C until use as an "AAV mutant barcode library". The viral genome quantity was measured using the same method as in Example 2-(4).

### Example 4: Mouse in vivo biodistribution analysis of AAV mutants using barcode sequences

### (1) Intravenous administration of AAV mutant barcode library to mice

The AAV mutant barcode library obtained in Example 3-(3) was administered to BALB/c mice at 7.5 x 10¹¹ vg via a tail vein. Four weeks after administration, the brain, spinal cord, lung, muscle, heart, and liver were removed. Genomic DNAs were extracted from these tissues using NucleoSpin (registered trademark) tissue (manufactured by Machley-Nagel). RNAs were also extracted from these tissues using RNeasy Plus Universal Mini (manufactured by Qiagen).

### (2) cDNA synthesis

Using the RNA extracted in Example 4-(1) as a template, cDNA was synthesized using PrimeScript RT reagent kit (manufactured by Takara Bio).

### (3) Amplification and purification of barcode sequence

Using the cDNA synthesized in Example 4-(2) as a template, an amplicon encoding the barcode region was amplified by PCR. In the PCR, a forward primer (SEQ ID NO: 7) and a reverse primer (SEQ ID NO: 8) were used as primers. The PCR was carried out under the conditions of preheating at 94°C for 1 minute, followed by 30 cycles of 98°C for 10 seconds, 55°C for 15 seconds, and 72°C for 60 seconds. After electrophoresis, the amplicons were purified using NucleoSpin (registered trademark) Gel and PCR Clean-up (manufactured by Machliner Gel).

### (4) Next-generation sequencing (NGS) analysis of barcode sequences

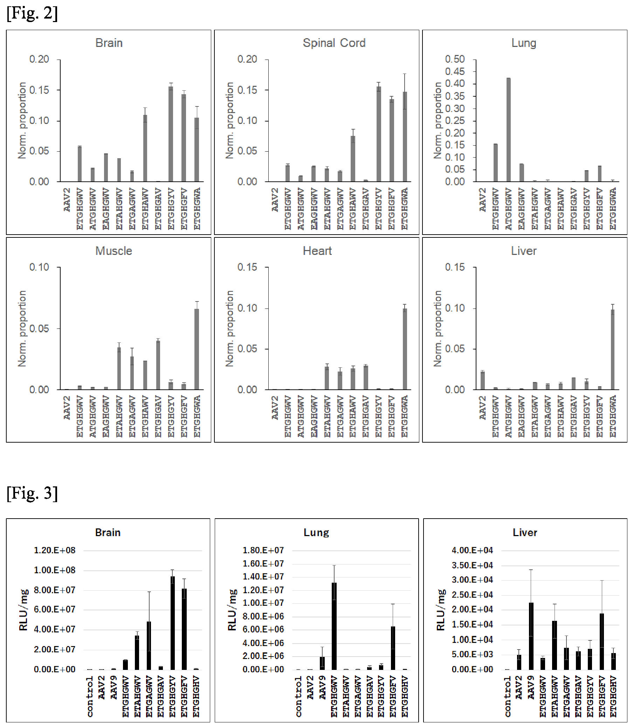

The amplicons purified in Example 4-(3) were subjected to NGS analysis using Miseq (manufactured by Illumina) to determine the appearance frequency of barcode sequences in each tissue. Furthermore, the gene transfer efficiency in each tissue was calculated as GFP/GAPDH by qPCR using the genomic DNA extracted in Example 4-(1) as a template. In the qPCR, Transgene Detection Primer set for real time (mouse) GFP-1 primer (manufactured by Takara Bio Inc.) was used to amplify the GFP sequence, and Mouse Housekeeping Gene Primer Set (manufactured by Takara Bio Inc.) was used to amplify the GAPDH sequence. The gene transfer efficiency was used to normalize the appearance frequency of the barcode sequence obtained by NGS analysis, and thereby the appearance frequency of the AAV vector in each tissue was calculated (Figure 2).

As shown in Figure 2, AAV vectors having ETGHAWV (SEQ ID NO: 3), ETGHGYV (SEQ ID NO: 1), ETGHGFV (SEQ ID NO: 2), and ETGHGWA (SEQ ID NO: 4) showed high infectivity for the brain and spinal cord. Particularly, AAV vectors having ETGHAWV (SEQ ID NO: 3), ETGHGYV (SEQ ID NO: 1) and ETGHGFV (SEQ ID NO: 2) showed high tropism for the brain and spinal cord.

### Example 5: Evaluation of AAV vectors having specific amino acid sequences

### (1) Preparation of pRC2Km-Insert plasmid

Multiple plasmids encoding Cap proteins containing various amino acid sequences inserted between amino acid numbers 588 and 589 of AAV2 Cap protein were prepared by modifying kanamycin-resistant plasmid pRC2Km (manufactured by Takara Bio Inc.) containing sequences encoding AAV2 Rep protein and AAV2 Cap protein. Finally obtained pRC2Km-Insert plasmids are as follows: pRC2Km-ETGHGWV, pRC2Km-ETAHGWV, pRC2Km-ETGAGWV, pRC2Km-ETGHGAV, pRC2Km-ETGHGYV, pRC2Km-ETGHGFV, and pRC2Km-ETGHGHV.

### (2) Preparation and purification of AAV vector

The 293T cells were transfected with pAAV-CAG-fLuc (manufactured by Takara Bio Inc.), pHelper Vector (manufactured by Takara Bio Inc.), and the pRC2Km-Insert plasmid obtained in Example 5-(1) using PEIpro (manufactured by Polyplus). As a control, the 293T cells were transfected with pRC2-mi342 Vector (manufactured by Takara Bio Inc.) or pRC9 Vector (manufactured by Takara Bio Inc.) instead of the pRC2Km-Insert plasmid. The transfected 293T cells were cultured in a CO₂ incubator at 37°C for 72 hours. Various AAV vectors produced were collected and purified using the same method as in Example 2-(3). For affinity purification of AAV9 vectors, POROS CaptureSelect^{™} AAV9 Affinity Resin (manufactured by Thermo) was used.

### (3) AAV vector infection by administration into the tail vein of mice

The AAV vector obtained in Example 5-(2) was administered to Balb/c mice (purchased from by SLC) via a tail vein at a dose of 1 x 10¹¹ vg/mouse. As a control, a solvent was administered. Thirty days after administration, the brain, lungs, and liver were removed. Protein was extracted from each tissue, and then subjected to measurement of luciferase activity using ONE-Glo Luciferase Assay System (manufactured by Promega). Furthermore, a relative light unit (RLU) per weight (mg) in each tissue was calculated (Figure 3).

As shown in FIG. 3, the AAV vectors having ETGHGYV (SEQ ID NO: 1) and ETGHGFV (SEQ ID NO: 2) showed high infectivity for the mouse brain tissue. Particularly, the AAV vector having ETGHGYV (SEQ ID NO: 1) showed high tropism for the mouse brain tissue.

Even when the amino acid residue at position 587 of the AAV2 Cap protein in the Cap protein encoded by each of the above pRC2Km-Insert plasmids was substituted with asparagine, results equivalent to or better than those shown in Figure 3 were obtained. Furthermore, when the amino acid residue at position 587 in the Cap protein containing ETGHGWV was substituted with asparagine (the amino acid sequence is shown in SEQ ID NO: 11), results equivalent to or better than those shown in Figure 3 were obtained.

### Example 6: Evaluation of AAV vector having specific amino acid sequence in retina

### (1) Preparation and purification of AAV vector

The 293T cells were transfected with pAAV-CAG-AcGFP (manufactured by Takara Bio Inc.), pHelper Vector (manufactured by Takara Bio Inc.), and the pRC2Km-Insert plasmid obtained in Example 5-(1) using PEIpro (manufactured by Polyplus). The transfected 293T cells were cultured in a CO₂ incubator at 37°C for 72 hours. Various AAV vectors produced were collected and purified using the same method as in Example 2-(3).

### (2) AAV vector infection by intravitreal administration in mice

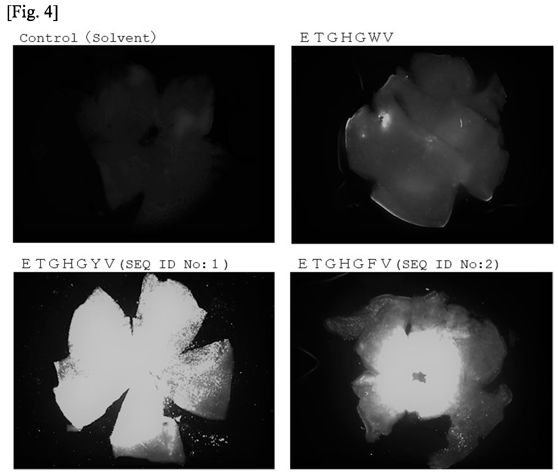

The AAV vectors containing ETGHGWV, ETGHGYV (SEQ ID NO: 1), and ETGHGFV (SEQ ID NO: 2) were administered intravitreally to Balb/c mice (purchased from SLC) at a dose of 4 x 10⁹ vg/eye. As a control, a solvent was administered. Retinas were removed 14 days after administration. GFP fluorescence in the retina was observed under a fluorescence microscope (Figure 4).

As shown in Figure 4, the AAV vectors having ETGHGYV (SEQ ID NO: 1) and ETGHGFV (SEQ ID NO: 2) showed higher infectivity for the mouse retina than the vector having ETGHGWV. Furthermore, the AAV vectors containing ETGHGYV (SEQ ID NO: 1) and ETGHGFV (SEQ ID NO: 2) showed higher infectivity for the mouse retina than the wild-type AAV2 vector (Data Not Shown).

The plasmids used to prepare the above-mentioned three types of AAV vectors were each modified to replace the amino acid residue at position 587 in the encoded Cap protein with asparagine. When a similar study to that described above was performed, results equivalent to or better than those described above were obtained.

### INDUSTRIAL APPLICABILITY

The present invention provides an AAV capsid protein mutant having high tropism for the brain and low tropism for the lung, and an amino acid sequence of the mutant, and provides a method for efficiently transferring a gene into the brain.

### SEQUENCE LISTING FREE TEXT

SEQ ID NO:1: Peptide sequence ETGHGYV for AAV capsid protein mutant
SEQ ID NO:2: Peptide sequence ETGHGFV for AAV capsid protein mutant
SEQ ID NO:3: Peptide sequence ETGHAWV for AAV capsid protein mutant
SEQ ID NO:4: Peptide sequence ETGHGWA for AAV capsid protein mutant
SEQ ID NO:5: Oligo DNA containing random 15 nucleotides
SEQ ID NO:6: Primer for synthesizing double strand DNA
SEQ ID NO:7: Forward primer for amplification of random peptide coding region
SEQ ID NO:8: Reverse primer for amplification of random peptide coding region
SEQ ID NO:9: Amino acid sequence of wild-type AAV2 VP1 capsid protein
SEQ ID NO:10: Amino acid sequence of AAV2(587Q) VP1 capsid protein
SEQ ID NO:11: Amino acid sequence of AAV2-ETGHGWV VP1 capsid protein
SEQ ID NO:12: Amino acid sequence of AAV2-ETGHGYV VP1 capsid protein

## Claims

1. A nucleic acid encoding a mutant of an adeno-associated virus (AAV) capsid protein which comprises a peptide comprising an amino acid sequence selected from the group consisting of SEQ ID NOs: 1-4.

2. The nucleic acid according to claim 1, wherein the AAV capsid protein is derived from AAV2.

3. The nucleic acid according to claim 2, wherein the peptide is placed at a position between amino acid number 588 and amino acid number 589 in VP1 of AAV2.

4. A recombinant DNA comprising the nucleic acid according to any one of claims 1 to 3.

5. A cell comprising the nucleic acid according to any one of claims 1 to 3.

6. An AAV particle comprising a mutant of an AAV capsid protein which comprises a peptide comprising an amino acid sequence selected from the group consisting of SEQ ID NOs: 1-4.

7. The AAV particle according to claim 6, wherein the AAV capsid protein is derived from AAV2.

8. The AAV particle according to claim 7, wherein the peptide is placed at a position between amino acid number 588 and amino acid number 589 in VP1 of AAV2.

9. A method of producing a gene-transduced cell, the method comprising a step of bringing an AAV particle comprising a mutant of an AAV capsid protein which comprises a peptide comprising an amino acid sequence selected from the group consisting of SEQ ID NOs: 1-4 into contact with a cell.

10. The method according to claim 9, wherein the AAV capsid protein is derived from AAV2.

11. The method according to claim 10, wherein the peptide is placed at a position between amino acid number 588 and amino acid number 589 in VP1 of AAV2.

12. A method of producing an AAV particle, the method comprising using the cell according to claim 5 as a host.
